(19) Europäisches Patentamt
European Patent Office
Office européen des brevets

(11) Veröffentlichungsnummer: **0 177 853**
**B1**

(12) # EUROPÄISCHE PATENTSCHRIFT

(45) Veröffentlichungstag der Patentschrift: **12.07.89**

(51) Int. Cl.⁴: **A61K 9/02**, A61K 31/54, A61K 47/00

(21) Anmeldenummer: **85112251.5**

(22) Anmeldetag: **27.09.85**

(54) **Antirheumatisch wirksame Suppositorien und Verfahren zu deren Herstellung.**

(30) Priorität: **29.09.84 DE 3435843**

(43) Veröffentlichungstag der Anmeldung: **16.04.86 Patentblatt 86/16**

(45) Bekanntmachung des Hinweises auf die Patenterteilung: **12.07.89 Patentblatt 89/28**

(84) Benannte Vertragsstaaten: **AT CH DE FR GB IT LI**

(56) Entgegenhaltungen:
**EP-A- 0 093 999**
**US-A- 4 233 299**

(73) Patentinhaber: **GÖDECKE AKTIENGESELLSCHAFT, Salzufer 16, D-1000 Berlin 10(DE)**

(72) Erfinder: **Knecht, Adolf, Dr., Kapellenweg 4a, D-7800 Freiburg(DE)**

EP 0 177 853 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Nicht-steroidale Antirheumatica werden üblicherweise nicht nur in oralen Darreichungsformen (Kapseln, Tabletten) in der Therapie verwendet, sondern auch in parenteralen und insbesondere rektalen Applikationsformen.

Zur Erzielung einer ausreichenden Bioverfügbarkeit bei oralen und rektalen Arzneizubereitungen ist die Geschwindigkeit, mit der ein Wirkstoff in Lösung geht, von entscheidender Bedeutung.

Die in der Therapie außerordentlich gut bewährten Substanzen mit dem Oxicam-Grundgerüst, wie z. B. Piroxicam oder Isoxicam zeichnen sich durch eine nur geringe Wasserlöslichkeit aus. Gleichzeitig liegt die zu verabreichende Einzeldosis relativ hoch, bei Isoxicam z. B. zwischen 100 mg und 300 mg, so daß es schwierig ist, galenisch die erforderliche Bioverfügbarkeit sicherzustellen.

Bei den oralen Formulierungen kann eine hohe Bioverfügbarkeit zwar durch geeignete Formulierungen gewährleistet werden. Die relative Bioverfügbarkeit in vivo ist gegenüber der parenteralen Applikationsform als Standard mit über 90 % außerordentlich hoch, wobei allerdings berücksichtigt werden muß, daß im gastrointestinalen Trakt nach oraler Verabreichung verhältnismäßig große Flüssigkeitsmengen zur Auflösung des Wirkstoffes zur Verfügung stehen.

Für rektale Applikationsformen liegen jedoch vollkommen andere Bedingungen vor. Isoxicam hat, in konventionellen Triglyceridmassen zu Zäpfchen verarbeitet, eine weit schlechtere Bioverfügbarkeit als zunächst erwartet werden kann. Auch Zusätze von oberflächenaktiven Substanzen oder die Verwendung von hydrophilen Trägermassen bewirken nur graduelle Verbesserungen der Bioverfügbarkeit. Es werden jedoch in keinem Falle Plasmaspiegelverläufe erreicht, die mit der oralen Verabreichung gleicher Dosen vergleichbar sind.

Dies kann mit der Tatsache erklärt werden, daß im Rektum normalerweise nur sehr begrenzte Mengen an Flüssigkeit zur Verfügung stehen und damit weitaus schlechtere Bedingungen zur Resorption gegeben sind, als im Gastrointestinaltrakt nach oraler Verabreichung.

Aus der DE-A1-3 217 315 ist bekannt, daß sich Isoxicam durch Verwendung von Methylglucamin in überstöchiometrischen Mengen für eine parenterale Applikationsform in stabile Lösungen bringen läßt, insbesondere wenn gleichzeitig ein mit Wasser mischbares organisches Lösungsmittel wie z. B. Polyethylenglykol zugesetzt wird. Die dieser DE-A1 entsprechende US-A- 4 233 299 führt sogar vom beanspruchten Gegenstand weg, weil aus Beispiel 3 ersichtlich ist, daß nicht erkannt wurde, daß sich die an sich bekannten (Oxicam-Mehylglucamin) Salze mit überraschendem Effekt auch besonders für Suppositorien-Einsatz eignen. Lösungen hingegen, die gemäß EP-A-2482 Methylglucamin im stöchiometrischen Verhältnis zum Wirkstoff enthalten, besitzen wenn Isoxicam eingesetzt wird, keine befriedigende Stabilität, weil wässrige Lösungen des stöchiometrischen Salzes nach kurzer Zeit Rekristallisation zeigen, wobei das schwer lösliche Isoxicam ausfällt. Im Hinblick auf die Verwendung des stöchiometrischen Salzes in rektalen Applikationsformen war damit zu erwarten, daß gegenüber den im Gastrointestinaltrakt vorliegenden Bedingungen schlechtere Verhältnisse - weniger verfügbares Flüssigkeitsvolumen zur Lösung der Wirkstoffmenge sowie anteilige Verminderung der Bioverfügbarkeit durch teilweise Ausfällung des schwerlöslichen Isoxicam im Rektum - vorliegen.

Es wurde nun überraschend gefunden, daß das stöchiometrische Salz aus Isoxicam und N-Methylglucamin, eingearbeitet in Triglycerid- Zäpfchenmassen, zu Zäpfchen verarbeitet werden kann, welche im In-vivo- Versuch am Menschen Bioäquivalenz gegenüber oralen Formen (Kapseln oder Tabletten) zeigen. Sowohl die Plasmaspiegelmaxima als auch die Flächen unter den Plasmaspiegelkurven weichen bei rektaler Verabreichung nicht signifikant von den für die orale Verabreichung gefundenen Werten ab.

Bei den sehr geringen Volumina an Flüssigkeit in einem menschlichen Rektum war es nicht vorherzusehen, daß aus dem Salz eines Wirkstoffes, der bei neutralem pH-Wert nur zu 0,04 mg/ml in Wasser löslich ist, pro Zeiteinheit gleich viel zur Resorption zur Verfügung gestellt werden kann, wie nach oraler Einnahme der gleichen Dosis des Wirkstoffes.

Somit ist es möglich, den extrem schwer löslichen Wirkstoff Isoxicam in eine rektale Verabreichungsform zu bringen, die mit gleicher Dosierung wie die etablierte orale Verabreichungsform in die Therapie eingeführt werden kann.

Gegenstand der vorliegenden Erfindung ist daher ein Suppositorium, bestehend aus einer üblichen Zäpfchenmasse und aus einem Salz eines antirheumatischen Oxicamderivats der Formel I

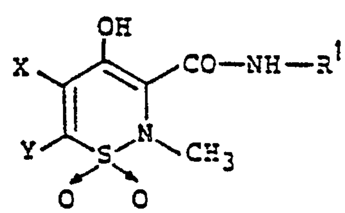

(I),

in welcher $R^1$ einen heterocyclischen Ring bedeutet und X zusammen mit Y einen ankondensierten aromatischen Ring darstellt, mit einer organischen Base, dadurch gekennzeichnet, daß als organische Base eine Verbindung der allgemeinen Formel II

$$R^2\text{-NH-CH}_2\text{-(CHOH)}_4\text{-CH}_2\text{OH} \qquad \text{(II)},$$

in welcher $R^2$ ein Wasserstoffatom oder eine Alkylgruppe mit 1 bis 6 Kohlenstoffatomen, insbesondere eine Methyl, Ethyl- oder Propylgruppe bedeutet, in stöchiometrischer Menge eingesetzt ist.

Unter Zäpfchenmasse versteht man sowohl hydrophile Substanzen, wie Seifengele, Glyceringelatine oder physiologisch verträgliche Polymere, z. B. Polyvinylpyrrolidone oder Polyethylenblykole, als auch hydrophobe Substanzen, wie Fette und deren Ester oder Alkohole und deren Ester, Talg, Wachse

oder Öle und deren hydrierte Fraktionen. Ausserden kommen Mischungen dieser Stoffe zur Anwendung, die auch weitere Zusätze, wie Metallseifen, Salze organischer Säuren oder Kohlenhydrate enthalten können.

Bevorzugt zur Herstellung der Zäpfchen werden Triglyceride verwendet.

Ein weiterer Gegenstand der Erfindung ist daher ein Verfahren zur Herstellung solcher Suppositorien, dadurch gekennzeichnet, dass man das Oxicamderivat der Formel I mit einer organischen Base zum Salz umsetzt, wobei als organische Base eine Verbindung der allgemeinen Formel II in stöchiometrischer Menge eingesetzt wird, und das Salz bei 30-70°C in die Zäpfchenmasse einarbeitet und diese zu Suppositorien ausformt und erkalten lässt, vorzugsweise, daß Isoxicam-Meglumin und Triglyceride bei ca. 50°C in einer Schmelze verarbeitet und temperiert auf ca. 35°C, zu Suppositorien gegossen werden, die 100 bis 300 mg Isoxicam enthalten.

Bevorzugt wird soviel Isoxicam-Mehlumin eingesetzt, dass die Zubereitung 322 mg pro Zäpfchen enthält. Das entspricht bei einem Wassergehalt von 2,06 % des Isoxicam-Methylglucamins einer Menge von 200 mg Isoxicam pro Suppositorium.

Die nachfolgenden Beispiele dienen zur näheren Erläuterung der Erfindung.

Beispiel 1

Zur Herstellung von 10.000 Suppositorien mit je 200 mg des Wirkstoffs Isoxicam werden 2,0 kg des feinkristallinen Wirkstoffs zusammen mit 4,0 kg Lactose in 15,0 kg Hartfett DAB, bei maximal 50° C geschmolzen, eingearbeitet. Danach wird die Mischung mittels einer Zahnkolloidmühle homogenisiert. Die Schmelze wird auf 35° C temperiert und in gekühlte Formen gegossen.

Zäpfchen dieser Zusammensetzung zeigten im vergleichenden Bioverfügbarkeitsversuch gegenüber Isoxicam Kapseln mit je 200 mg Wirkstoff eine durchschnittliche relative Bioverfügbarkeit von nur ca. 50 %.

Beispiel 2

Zur Herstellung von 10.000 erfindungsgemäßen Zäpfchen werden 3,22 kg Isoxicam-Meglumin, gemahlen, zusammen mit 950 g Neutralöl in 15,7 kg Hartfett DAB, geschmolzen, eingearbeitet. Der Ansatz wird mit Hilfe einer Zahnkolloidmühle homogenisiert, auf ca. 36° C abgekühlt und in gekühlte Formen zu Zäpfchen ausgegossen. Diese Zäpfchen enthalten je 322 mg Isoxicam-Meglumin (Wassergehalt 2,06 %) entsprechend 200 mg Isoxicam.

In einem vergleichenden Bioverfügbarkeitsversuch zeigten diese Zäpfchen gegenüber 200 mg oral verabreichtes Isoxicam vergleichbare Plasmaspiegel-Maxima und vergleichbare Flächen unter den Plasmaspiegel- Kurven.

**Patentansprüche**

1. Suppositorien, bestehend aus einer üblichen Zäpfchenmasse und aus einem Salz eines antirheumatischen Oxicamderivats der Formel I

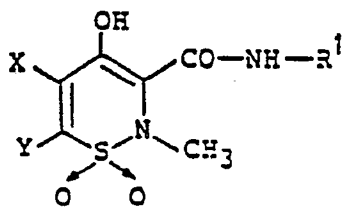

(I),

in welcher R1 einen heterocyclischen Ring bedeutet und X zusammen mit Y einen ankondensierten aromatischen Ring darstellt, mit einer organischen Base, dadurch gekennzeichnet, daß als organische Base eine Verbindung der allgemeinen Formel II

$$R^2\text{-NH-CH}_2\text{-(CHOH)}_4\text{-CH}_2\text{OH} \qquad \text{(II)}$$

in welcher R2 ein Wasserstoffatom oder eine Alkylgruppe mit 1 bis 6 Kohlenstoffatomen, insbesondere eine Methyl, Ethyl- oder Propylgruppe bedeutet, in stöchiometrischer Menge eingesetzt ist.

2. Suppositorien mit einem Gehalt an mindestens einem Salz nach Anspruch 1, dadurch gekennzeichnet, daß diese, bezogen auf 1 Mol der Verbindung der allgemeinen Formel I, exakt 1 Mol der Verbindung der allgemeinen Formel II enthalten.

3. Suppositorien nach Anspruch 1 und 2, dadurch gekennzeichnet, daß sie als Verbindug der Formel I Isoxicam enthalten.

4. Suppositorien nach Anspruch 1 bis 3, dadurch gekennzeichnet, daß sie als Base der Formel II Methylglucamin enthalten.

5. Suppositorien nach Anspruch 1 bis 4, dadurch gekennzeichnet, daß sie als Zäpfchenmasse Triglyceride enthalten.

6. Suppositorien nach Anspruch 1 bis 5, dadurch gekennzeichnet, daß sie 100 bis 300 mg Wirkstoff gemäß Formel I enthalten.

7. Verfahren zur Herstellung von Suppositorien nach Anspruch 1, dadurch gekennzeichnet, daß man das Oxicamderivat der Formel I mit einer organischen Base zum Salz umsetzt, wobei als organische Base eine Verbindung der allgemeinen Formel II in stöchiometrischer Menge eingesetzt wird, und das Salz bei 30–70° in die Zäpfchenmasse einarbeitet und diese zu Suppositorien ausformt und erkalten läßt.

8. Verfahren gemäß Anspruch 7, dadurch gekennzeichnet, daß als Oxicamderivat I Isoxicam und als Base II Methylglucamin im Molverhältnis 1:1 eingesetzt wird.

9. Verfahren gemäß Anspruch 7 und 8, dadurch gekennzeichnet, daß als Zäpfchenmasse Triglyceride verwendet werden.

10. Verfahren gemäß Anspruch 7 und 8, dadurch gekennzeichnet, daß Isoxicam-Meglumin und Triglyceride bei ca. 50°C ein einer Schmelze verarbeitet und temperiert auf ca. 35°C, zu Suppositorien gegossen werden, die 100 bis 300 mg Isoxicam enthalten.

**Claims**

1. Suppositories, comprising a usual suppository base and a salt of an antirheumatic oxicam derivat of formula I

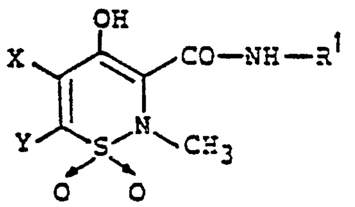

(I),

in which $R^1$ represents a heterocyclic ring and X together with Y a fused aromatic ring with an organic base, characterized in that a compound of general formula II

$$R^2\text{-NH-CH}_2\text{-(CHOH)}_4\text{-CH}_2\text{OH} \qquad \text{(II)},$$

in which $R^2$ represents a hydrogen atom or an alkyl group with 1 to 6 carbon atoms, in particular methyl, ethyl of propyl group, is used in stoichiometric proportion as the organic base.

2. Suppositories containing at least one salt according to Claim 1, characterized in that they contain, relative to 1 mole of the compound of general formula I, exactly 1 mole of the compound of general formula II.

3. Suppositories as in Claim 1 or 2, characterized in that they contain isoxicam as a compound of formula I.

4. Suppositories as in Claim 1 to 3, characterized in that they contain methylglucamine as a base of formula II.

5. Suppositories as in Claim 1 to 4, characterized in that they contain triglyceride as a suppository base.

6. Suppositories as in Claim 1 to 5, characterized in that they contain 100 to 300 mg of the active ingredient according to formula I.

7. Process for the preparation of suppositories according to Claim 1, characterized in that the oxicam derivative of formula I with an organic base is converted to the salt, a compound of general formula II being used in stoichiometric proportion as an organic base, and the salt is incorporated at 30–70°C in the suppository base, which is moulded into suppositories and allowed to cool.

8. Process according to Claim 7, characterized in that isoxicam is used as the oxicam derivative I and methylglucamine is used as the base II, in the molar ratio 1 : 1.

9. Process according to Claim 7 and 8, characterized in that triglycerides are used as the suppository base.

10. Process according to Claims 7 to 9, characterized in that isoxicam-meglumine and triglycerides are worked up at about 50°C in a molten state and, tempered to approximately 35°C, moulded into suppositories containing 100 to 300 mg of isoxicam.

**Revendications**

1. Suppositoires formés d'une masse des constituants habituellement employés à cette fin et d'un sel d'un composé antirhumatismal dérivé de l'oxicam répondant à la formule I

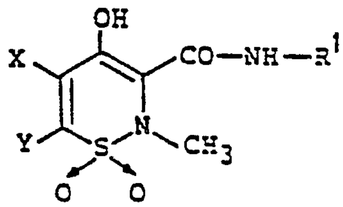

(I),

dans laquelle: $R^1$ est un noyau hétérocyclique; et X et Y représentent un cycle aromatique condensé audit noyau, une base organique, caractérisés en ce que l'on greffe, comme base organique, en proportion stoechiométrique, un dérivé répondant à la formule générale II;

$$R^2\text{-NH-CH}_2\text{-(CHOH)}_4\text{-CH}_2\text{OH} \qquad \text{(II)},$$

dans laquelle: $R^2$ est un atome d'hydrogène ou un groupe alkyle ayant 1 à 6 atomes de carbone, en particulier un groupe méthylique, éthylique ou propylique.

2. Suppositoires contenant au moins un sel selon la revendication 1, caractérisés en ce qu'ils contiennent exactement une mole du dérivé de formule générale II par mole de la liaison de formule générale I.

3. Suppositoires selon la revendication 1 ou la revendication 2, caractérisés en ce qu'ils contiennent en tant que dérivé de formule I de l'isoxicam.

4. Suppositoires selon les revendication 1 à 3, caractérisés en ce qu'ils contiennent comme base formule II de la méthylalucamine.

5. Suppositoires selon les revendications 1 à 4, caractérisés en ce qu'ils contiennent dans leur masse du triglycéride.

6. Suppositoires selon les revendications 1 à 5, caractérisés en ce qu'ils contiennent 100 à 300 mg de substance active de formule I.

7. Procédé pour la fabrication de suppositoires selon la revendication 1, caractérisé en ce que l'on transforme en sel, par réaction avec une base organique, le dérivé d'oxicam de formule I et en ce que, en tant que base organique, on incorpore, en quantité stoechiométrique, un dérivé de formule générale II et en ce qu'ensuite le sel est trituré entre 30 et 70°C, dans la masse destinée à former les suppositoires, laquelle est mise sous forme de suppositoires et refroidie.

8. Procédé selon la revendication 7, caractérisé en ce que le dérivé d'oxicam est l'isoxicam et que la méthylglucamine est la base II, utilisés dans une proportion molaire 1/1.

9. Procédé selon les revendications 7 et 8, caractérisé en ce que le triglycéride forme un constituant de base du suppositoire.

10. Procédé selon les revendications 7 à 9, caractérisé en ce que l'isoxicam méglumine et le triglycéride sont triturés à environ 50°C à l'état fondu, et ramenés à environ 35°C pour être coulés sous forme de suppositoires qui contiennent 100 à 300 mg d'isoxicam.